# EUROPEAN PATENT SPECIFICATION

(11) **EP 2 813 514 B1**
(45) Date of publication and mention of the grant of the patent: **02.11.2016**
(21) Application number: 13746833.6
(22) Date of filing: 29.01.2013
(51) Int. Cl.: C07K 14/605, B01D 15/18

(54) **METHOD FOR PURIFYING SOLID-PHASE SYNTHETIC CRUDE LIRAGLUTIDE**
VERFAHREN ZUR REINIGUNG VON SYNTHETISCHEM FESTPHASEN-ROHLIRAGLUTID
PROCÉDÉ DE PURIFICATION DU LIRAGLUTIDE BRUT SYNTHÉTIQUE EN PHASE SOLIDE

(30) Priority: 10.02.2012 CN 201210029818
(43) Date of publication of application: 17.12.2014
(73) Proprietor: Hybio Pharmaceutical Co., Ltd., Shenzhen, Guangdong 518057 (CN)
(72) Inventor: QIN, Liangzheng, Shenzhen Guangdong 518057 (CN); PAN, Junfeng, Shenzhen Guangdong 518057 (CN); MA, Yaping, Shenzhen Guangdong 518057 (CN); YUAN, Jiancheng, Shenzhen Guangdong 518057 (CN)
(74) Representative: HGF Limited
(86) International application number: PCT/CN2013/071063
(87) International publication number: WO 2013/117135

(56) References cited:
- WO-A1-00/34332
- WO-A2-2006/091231
- WO-A2-2012/006598
- CN-A- 101 208 099
- CN-A- 101 627 966
- CN-A- 102 085 355
- CN-A- 102 321 170
- US-A1- 2011 313 131
- KNUDSEN, L.B. ET AL.: 'Potent Derivatives of Glucagon-like Peptide-I with Pharmacokinetic Properties Suitable for Once Daily Administration.' J. MED. CHEM. vol. 43, no. 9, April 2000, pages 1664 - 1669, XP002222050

## Description

### Field of the invention

The present invention relates to the field of bio-pharmaceuticals, and in particular, to a method for purifying solid-phase synthetic crude liraglutide.

### Background of the invention

Diabetes Mellitus (DM) is a worldwide common disease with high incidence rate. Based on the data recently published by the WHO (world health organization), there have been as many as 180 million diabetic patients all over the world in 2007, with an increasing incidence enhanced year by year. According to epidemiological statistics, there have been almost 92 million diabetic patients in China in 2010. Since every individual system in the body is involved in diabetes which may even induce fatal complications. Diabetes has a strong impact on the labor capacity of human beings and threatens their life safety, resulting in great damage to the health of human beings. Diabetes is primarily divided into type I and type II diabetes, in which the latter accounts for more than 90% in total diabetic patients.

Liraglutide is a glicetin 1 (GLP-1) analog used for long-acting treatment of type II diabetes, which is a kind of GLP-1 receptor agonists, and is the first human glicetin 1 (GLP-1) analog developed for the treatment of type II diabetes. Liraglutide is developed by Novo Nordisk, and approved for marketing by FDA on January 25, 2010, and approved by SFDA on March 4, 2011. As a new generation of hypoglycemic agent based on incretin, liraglutide not only has long acting duration, but also retains multiple biological activities of natural GLP-1, which is safety and effective in lowering blood sugar, and may be used for protection from a plurality of cardiovascular risk factors, resulting in a new choice for the treatment of type II diabetes. The clinical therapeutic effect of liraglutide is encouraging.

At present, China is totally dependent upon importation from foreign countries for liraglutide, which makes it expensive. Liraglutide is produced by Novo Nordisk via genetic recombination technology. Solid-phase chemical synthesis of polypeptide is an important technical means in the field of research and production of polypeptide and protein pharmaceuticals due to advantages such as oriented synthesis and less solvent consumption. However, impurities produced in the chemical synthesis are difficult to be separated and purified because of its similar properties, which make the purification technique become one of the bottlenecks in the process, leading to difficulty in its industrialization.

At present, a RP-HPLC method has been reported for the purification of liraglutide (Journal of Medicinal Chemistry 43, 1664-1669, 2000) using a cyanopropyl column (Zorbax 300SB-CN) with a standard TFA/acetonitrile system (a concentration gradient of acetonitrile from 0 to 100% within 60 min) as mobile phase at a column temperature of 65°C, leading to a yield of 35% for the target product; the same method has also been adopted in Chinese patent 200610110898.3 and 200510107588, with a purification yield of 28%.

The purification of liraglutide is difficult due to its long peptide chain and high hydrophobicity resulting from the presence of palmityl group. In the present invention, a purification method for liraglutide is provided obtained by solid phase chemical synthesis, which results in high purity and yield, and can be readily industrialized.

### Summary of the invention

On such a basis, a method for purifying crude liraglutide obtained by solid-phase synthesis is provided in the present invention. In this method, crude liraglutide solution is obtained by dissolving the crude peptide from solid-phase synthesis in aqueous acetonitrile solution, which is then subjected to four-step HPLC purification to obtain purified liraglutide with high purity and yield.

In order to achieve the object of the present invention, a technical embodiment is provided below:
A method for purifying crude liraglutide obtained from solid-phase synthesis, which is characterized by comprising the following steps:
   Step 1: a solution of crude liraglutide is obtained by dissolving crude liraglutide obtained from solid-phase synthesis in aqueous acetonitrile solution;
   Step 2: the solution of crude liraglutide is subjected to a first HPLC purification using octylsilane bonded silica as stationary phase, and using aqueous isopropanol solution containing 0.1-0.2% trifluoroacetic acid as mobile phase A and acetonitrile containing 0.1-0.2% trifluoroacetic acid as mobile phase B eluting at a linear gradient from 20-40% B to 40-60% B, and target peak is collected as the first fraction;
   Step 3: the first fraction is subjected to a second HPLC purification using cyanosilane bonded silica as stationary phase, and using 0.05-0.15% (mass concentration) aqueous perchloric acid solution as mobile phase A and 0.05-0.15% (mass concentration) perchloric acid in acetonitrile as mobile phase B eluting at a linear gradient from 40% B to 70% B, and target peak is collected as the second fraction;
   Step 4: the second fraction is subjected to a third HPLC purification using octylsilane bonded silica as stationary phase, and using 0.01-0.06% (mass concentration) aqueous ammonia solution as mobile phase A and acetonitrile of chromatographic grade as mobile phase B eluting at a linear gradient from 30% B to 60% B, and target peak is collected as the third fraction;
   Step 5: purified peptide is obtained from the third fraction by rotatory evaporation under reduced presser and lyophilization.

In the solid-phase synthesis of polypeptides, the purities are mainly short oligonucleoside fragments, salts and various protective groups, wherein, the impurities required for removal is mainly the default peptide and racemic peptide. In the present invention, the purity of the crude peptide from solid-phase synthesis is in the range from 50 to 60%, with the maximum impurity content of 5 to 8%.

Preferably, the volume ratio between acetonitrile and water in the aqueous acetonitrile solution is 10-30 : 70-90.

Preferably, the volume ratio between isopropanol and water in the aqueous isopropanol solution is 15-35 : 65-85.

Preferably, the flow rate for the first, second or third HPLC purification is 55-2000 ml/min in Step 2, 3, or 4, respectively.

Preferably, the flow rate for the first, second or third HPLC purification is 55-500 ml/min in Step 2, 3, or 4, respectively.

Preferably, the duration for the linear gradient elution in Step 2 or 3 is 40 min.

Preferably, the duration for the linear gradient elution in Step 4 is 30 min.

Preferably, the concentration after rotatory evaporation under reduced pressure in Step 5 is 50-70 mg/ml.

Preferably, the solid-phase synthesis is carried out by the following steps: in the presence of activating agent system, coupling the solid phase support resin with N-terminal Fmoc-protected glycine to obtain Fmoc-Gly-resin; according to the backbone sequence of liraglutide, sequentially coupling the amino acids with N-terminal Fmoc protection and side chain protection using solid-phase synthesis method, with Alloc protection for the side chain protection of lysine; removing the protective group Alloc from the side chain of lysine; coupling palmitoyl-Gllu-OtBu to the side chain of lysine by solid-phase synthesis method; obtaining crude liraglutide after cleavage, and removal of protective group and resin.

A method for purifying crude liraglutide obtained by solid-phase synthesis is provided in the present invention. In this method, after dissolving the crude liraglutide from solid-phase synthesis in aqueous acetonitrile solution, the prepared solution is subjected to 4 steps of HPLC purification to obtain purified liraglutide with a yield of 61.1-64.4% and a purity of 98.2-98.7%.

### Detailed description of the invention

A method for purifying crude liraglutide obtained by solid-phase synthesis is disclosed by the present invention, which can be implemented by properly modifying the processing parameters by those skilled in the art with reference to the contents herein. Particularly, it should be noted that all similar replacements and modifications are apparent to those skilled in the art, all of which are regarded to be included in the present invention. The method of the present invention and the applications thereof have been described by preferred Examples.

All of the reagents used in the method provided herein for purification of liraglutide obtained by solid-phase synthesis are commercially available from related companies.

The present invention will be further illustrated with reference to the following Examples:

### Example 1

Liraglutide was obtained by solid-phase synthesis with a purity of 50%.

### Sample treatment:

2.2 g crude liraglutide was completely dissolved in 10% acetonitrile/90% water (V/V) with assistance of ultrasound, and subsequently the solution was filtered by a filter membrane and collected for future use.

### The first HPLC purification:

Conditions for purification: chromatographic column: a column using octylsilane bonded silica as the stationary phase, with its diameter and length of 50 mm×250 mm. Mobile phase: phase A: 0.1% TFA in 85% water/15% aqueous isopropanol solution; phase B: 0.1% TFA in acetonitrile; flow rate: 55 ml/min; gradient: 40% B-60% B; detection wavelength: 275nm. The loading amount was 2.2 g.

Purification process: the column was loaded with sample after washing by aqueous acetonitrile solution with a concentration of 50% or more and equilibration, and the loading amount was 2.2 g. A fraction with purity greater than 95% was obtained by eluting with a linear gradient for 40 min and collected as target peak. The fraction of the target peak collected was concentrated to about 20 mg/ml by rotatory evaporation under reduced pressure at a temperature not higher than 35°C, and the resulting concentrate was used as the sample for the second purification.

### The second HPLC purification:

Conditions for purification: chromatographic column: the column using cyanosilane bonded silica as the stationary phase, with its diameter and length of 50 mm×250 mm. Mobile phase: phase A: 0.15% aqueous perchloric acid solution; phase B: 0.15% perchloric acid in acetonitrile; gradient: 40% B-70% B; detection wavelength: 275 nm. The loading amount was 1.2 g.

Purification process: the column was loaded with the fraction obtained by the first purification after washing by aqueous acetonitrile solution with a concentration of 50% or more and equilibration, and the loading amount was 1.2 g. A fraction with purity greater than 97% was obtained by eluting with a linear gradient for 40 min and collecting the target peak. The fraction of the target peak collected was concentrated to about 20 mg/ml by rotatory evaporation under reduced pressure at a temperature not higher than 35°C, and the resulting concentrate was used as the sample for the third purification for desalinization.

### The third HPLC purification for desalinization:

Chromatographic column: the column using octylsilane bonded silica as the stationary phase, with its diameter and length of 50 mm×250 mm. Phase A: 0.01% aqueous ammonia solution; phase B: acetonitrile of chromatographic grade; gradient: 30% B-60% B; detection wavelength: 275 nm. The loading amount was 1.0 g.

Purification process: the column was loaded with the fraction obtained by the second purification after washing by aqueous acetonitrile solution with a concentration of 50% or more and equilibration, and the loading amount was 1.0 g. A fraction with purity greater than 98% was obtained by eluting with a linear gradient for 30 min and collecting the target peak. The fraction of the target peak collected was concentrated to about 50 mg/ml by rotatory evaporation under reduced pressure at a temperature not higher than 35°C, and then lyophilized, resulting in 0.85 g active pharmaceutical ingredient liraglutide with a purity of 98.6% and an overall yield of 64.4%.

### Example 2

Liraglutide was prepared by solid-phase synthesis according to the following steps: in the presence of activating agent system, coupling solid-phase support resin with N-terminal Fmoc-protected glycine to obtain Fmoc-Gly-resin; according to backbone sequence of liraglutide, sequentially coupling amino acids with N-terminal Fmoc protection and side chain protection using solid-phase synthesis method, with Alloc protection for the side chain of lysine; removing the protective group Alloc from the side chain of lysine; coupling Palmitoyl-Gllu-OtBu to the side chain of lysine by solid-phase synthesis method; obtaining crude liraglutide after cleavage, and removal of protective group and resin. The purity of the crude peptide was 60%.

### Sample treatment:

2.5 g crude liraglutide was completely dissolved in 20% acetonitrile/80% water (V/V) with assistance of ultrasound, and subsequently the solution was filtered by a filter membrane and collected for future use.

### The first HPLC purification:

Conditions for purification: chromatographic column: the column using octylsilane bonded silica as the stationary phase, with its diameter and length of 50 mm×250 mm. Mobile phase: phase A: 0.2% trifluoroacetic acid in 75% water/25% isopropanol aqueous solution; phase B: 0.2% trifluoroacetic acid in acetonitrile; flow rate: 80 ml/min; gradient: 35% B-55% B; detection wavelength: 275 nm. The loading amount was 2.5 g.

Purification process: the column was loaded after washing by aqueous acetonitrile solution with a concentration of 50% or more and equilibration, and the loading amount was 2.5 g. A fraction with purity greater than 95% was obtained by eluting with a linear gradient for 40 min and collecting the target peak. The fraction of the target peak collected was concentrated to about 25 mg/ml by rotatory evaporation under reduced pressure at a temperature not higher than 35°C, and the resulting concentrate was used as the sample for the second purification.

### The second HPLC purification:

Conditions for purification: chromatographic column: the column using cyanosilane bonded silica as the stationary phase, with its diameter and length of 50 mm×250 mm. Mobile phase: phase A: 0.1% aqueous perchloric acid solution; phase B: 0.1% perchloric acid in acetonitrile; gradient: 40% B-70% B; detection wavelength: 275 nm. The loading amount was 1.4 g.

Purification process: the column was loaded with the fraction obtained by the first purification after washing by aqueous acetonitrile solution with a concentration of 50% or more and equilibration, and the loading amount was 1.4 g. A fraction with purity greater than 97% was obtained by eluting with a linear gradient for 40 min and collecting the target peak. The fraction of the target peak collected was concentrated to about 20 mg/ml by rotatory evaporation under reduced pressure at a temperature not higher than 35°C, and the resulting concentrate was used as the sample for the third purification for desalinization.

The third HPLC purification for desalinization: chromatographic column: the column using octylsilane bonded silica as the stationary phase, with its diameter and length of 50 mm×250 mm. Phase A: 0.04% aqueous ammonia solution; phase B: acetonitrile of chromatographic grade; gradient: 30% B-60% B; detection wavelength: 275 nm. The loading amount was 1.18 g.

Purification process: the column was loaded with the fraction obtained by the second purification after washing by aqueous acetonitrile solution with a concentration of 50% or more and equilibration, and the loading amount was 1.18 g. A fraction with purity greater than 98% was obtained by eluting with a linear gradient for 30 min and collecting the target peak. The fraction of the target peak collected was concentrated to about 60 mg/ml by rotatory evaporation under reduced pressure at a temperature not higher than 35°C, and then lyophilized, resulting in 0.92 g active pharmaceutical ingredient liraglutide with a purity of 98.4% and an overall yield of 61.3%.

### Example 3

Liraglutide was obtained by solid-phase synthesis with a crude peptide purity of 58%.

### Sample treatment:

3.0 g crude liraglutide was completely dissolved in 30% acetonitrile/70% water (V/V) with assistance of ultrasound, and subsequently the solution was filtered by a filter membrane and collected for future use.

### The first HPLC purification:

Conditions for purification: chromatographic column: the column using octylsilane bonded silica as the stationary phase, with its diameter and length of 50 mm×250 mm. Mobile phase: phase A: 0.2% TFA in 65% water/35% isopropanol aqueous solution; phase B: 0.2% TFA in acetonitrile; flow rate: 70 ml/min; gradient: 30% B-50% B; detection wavelength: 275 nm. The loading amount was 3.0 g.

Purification process: the column was loaded after washing by aqueous acetonitrile solution with a concentration of 50% or more and equilibration, and the loading amount was 3.0 g. A fraction with purity greater than 95% was obtained by eluting with a linear gradient for 40 min and collecting the target peak. The fraction of the target peak collected was concentrated to about 10 mg/ml by rotatory evaporation under reduced pressure at a temperature not higher than 35°C, and the resulting concentrate was used as the sample for the second purification.

### The second HPLC purification:

Conditions for purification: chromatographic column: the column using cyanosilane bonded silica as the stationary phase, with its diameter and length of 50 mm×250 mm. Mobile phase: phase A: 0.05% aqueous perchloric acid solution; phase B: 0.05% perchloric acid in acetonitrile; gradient: 40% B-70% B; detection wavelength: 275 nm. The loading amount was 1.53 g.

Purification process: the column was loaded with the fraction obtained by the first purification after washing by aqueous acetonitrile solution with a concentration of 50% or more and equilibration, and the loading amount was 1.53 g. A fraction with purity greater than 97% was obtained by eluting with a linear gradient for 40 min and collecting the target peak. The fraction of the target peak collected was concentrated to about 15 mg/ml by rotatory evaporation under reduced pressure at a temperature not higher than 35°C, and the resulting concentrate was used as the sample for the third purification for desalinization.

The third HPLC purification for desalinization: chromatographic column: the column using octylsilane bonded silica as the stationary phase, with its diameter and length of 50 mm×250 mm. Phase A: 0.06% aqueous ammonia solution; phase B: acetonitrile of chromatographic grade; gradient: 30% B-60% B; detection wavelength: 275 nm. The loading amount was 1.24 g.

Purification process: the column was loaded with the fraction obtained by the second purification after washing by aqueous acetonitrile solution with a concentration of 50% or more and equilibration, and the loading amount was 1.24 g. A fraction with purity greater than 98% was obtained by eluting with a linear gradient for 30 min and collecting the target peak. The fraction of the target peak collected was concentrated to about 70 mg/ml by rotatory evaporation under reduced pressure at a temperature not higher than 35°C, and then lyophilized, resulting in 1.1 g active pharmaceutical ingredient liraglutide with a purity of 98.7% and an overall yield of 61.1%.

### Example 4

Liraglutide was prepared by solid-phase synthesis according to the following steps: in the presence of activating agent system, coupling solid-phase support resin with N-terminal Fmoc-protected glycine to obtain Fmoc-Gly-resin; according to backbone sequence of liraglutide, sequentially coupling amino acids with N-terminal Fmoc protection and side chain protection using solid-phase synthesis method, with Alloc protection for the side chain of lysine; removing the protective group Alloc from the side chain of lysine; coupling palmitoyl-Gllu-OtBu to the side chain of lysine by solid-phase synthesis method; obtaining crude liraglutide after cleavage, and removal of protective group and resin. The purity of the crude peptide was 53%.

### Sample treatment:

25 g crude liraglutide was completely dissolved in 20% acetonitrile/80% water (V/V) with assistance of ultrasound, and subsequently the solution was filtered by a filter membrane and collected for future use.

### The first HPLC purification:

Conditions for purification: chromatographic column: the column using octylsilane bonded silica as the stationary phase, with its diameter and length of 150 mm×250 mm. Mobile phase: phase A: 0.1% TFA in 20% isopropanol/80% water; phase B: 0.1% trifluroacetic acid in acetonitrile; flow rate: 500 ml/min; gradient: 30% B-50% B; detection wavelength: 275 nm. The loading amount was 25 g.

Purification process: the column was loaded with sample after washing by aqueous acetonitrile solution with a concentration of 50% or more and equilibration, and the loading amount was 25 g. A fraction with purity greater than 95% was obtained by eluting with a linear gradient for 40 min and collecting the target peak. The fraction of the target peak collected was concentrated to about 20 mg/ml by rotatory evaporation under reduced pressure at a temperature not higher than 35°C, and the resulting concentrate was used as the sample for the second purification.

### The second HPLC purification:

Conditions for purification: chromatographic column: the column using cyanosilane bonded silica as the stationary phase, with its diameter and length of 150 mm×250 mm. Mobile phase: phase A: 0.15% aqueous perchloric acid solution; phase B: 0.15% perchloric acid in acetonitrile; flow rate: 500 ml/min; gradient: 40% B-70% B; detection wavelength: 275 nm. The loading amount was 12.25 g.

Purification process: the column was loaded with the fraction obtained by the first purification after washing by aqueous acetonitrile solution with a concentration of 50% or more and equilibration, and the loading amount was 12.25 g. A fraction with purity greater than 97% was obtained by eluting with a linear gradient for 40 min and collecting the target peak. The fraction of the target peak collected was concentrated to about 20 mg/ml by rotatory evaporation under reduced pressure at a temperature not higher than 35°C, and the resulting concentrate was used as the sample for the third purification for desalinization.

The third HPLC purification for desalinization: chromatographic column: the column using octylsilane bonded silica as the stationary phase, with its diameter and length of 150 mm×250 mm. Phase A: 0.05% aqueous ammonia solution; phase B: acetonitrile of chromatographic grade; flow rate: 500 ml/min; gradient: 30% B-60% B; detection wavelength: 275 nm. The loading amount was 10.7 g.

Purification process: the column was loaded with the fraction obtained by the second purification after washing by aqueous acetonitrile solution with a concentration of 50% or more and equilibration, and the loading amount was 10.7 g. A fraction with purity greater than 98% was obtained by eluting with a linear gradient for 30 min and collecting the target peak. The fraction of the target peak collected was concentrated to about 50 mg/ml by rotatory evaporation under reduced pressure at a temperature not higher than 35°C, and then lyophilized, resulting in 9.2 g active pharmaceutical ingredient liraglutide with a purity of 98.4% and an overall yield of 61.3%.

### Example 5

Liraglutide was obtained by solid-phase synthesis with a crude peptide purity of 56%.

### Sample treatment:

90 g crude peptide was completely dissolved in 30% acetonitrile/70% water (V/V) with assistance of ultrasound, and subsequently the solution was filtered by a filter membrane and collected for future use.

### The first HPLC purification:

Conditions for purification: chromatographic column: the column using octylsilane bonded silica as the stationary phase, with its diameter and length of 300 mm×250 mm. Mobile phase: phase A: 0.15% trifluoroacetic acid in 20% isopropanol/80% water; phase B: 0.15% trifluoroacetic acid in acetonitrile; flow rate: 2000 ml/min; gradient: 30% B-50% B; detection wavelength: 275 nm. The loading amount was 90 g.

Purification process: the column was loaded with sample after washing by aqueous acetonitrile solution with a concentration of 50% or more and equilibration, and the loading amount was 90 g. A fraction with purity greater than 95% was obtained by eluting with a linear gradient for 40 min and collecting the target peak. The fraction of the target peak collected was concentrated to about 25 mg/ml by rotatory evaporation under reduced pressure at a temperature not higher than 35°C, and the resulting concentrate was used as the sample for the second purification.

### The second HPLC purification:

Conditions for purification: chromatographic column: the column using cyanosilane bonded silica as the stationary phase, with its diameter and length of 300 mm×250 mm. Mobile phase: phase A: 0.15% aqueous perchloric acid solution; phase B: 0.15% perchloric acid in acetonitrile; flow rate: 2000 ml/min; gradient: 40% B-70% B; detection wavelength: 275 nm. The loading amount was 46 g.

Purification process: the column was loaded with the fraction obtained by the first purification after washing by aqueous acetonitrile solution with a concentration of 50% or more and equilibration, and the loading amount was 46 g. A fraction with purity greater than 97% was obtained by eluting with a linear gradient for 40 min and collecting the target peak. The fraction of the target peak collected was concentrated to about 20 mg/ml by rotatory evaporation under reduced pressure at a temperature not higher than 35°C, and the resulting concentrate was used as the sample for the third purification for desalinization.

The third HPLC purification for desalinization: chromatographic column: the column using octylsilane bonded silica as the stationary phase, with its diameter and length of 300 mm×250 mm. Phase A: 0.05% aqueous ammonia solution; phase B: acetonitrile of chromatographic grade; flow rate: 2000 ml/min; gradient: 30% B-60% B; detection wavelength: 275 nm. The loading amount was 40.1 g.

Purification process: the column was loaded with the fraction obtained by the second purification after washing by aqueous acetonitrile solution with a concentration of 50% or more and equilibration, and the loading amount was 40.1 g. A fraction with purity greater than 98% was obtained by eluting with a linear gradient for 30 min and collecting the target peak. The fraction of the target peak collected was concentrated to about 50 mg/ml by rotatory evaporation under reduced pressure at a temperature not higher than 35°C, and then lyophilized, resulting in 34.0 g active pharmaceutical ingredient liraglutide with a purity of 98.2% and an overall yield of 62.9%.

Methods provided by the present invention for purifying crude liraglutide obtained by solid-phase synthesis have been described in detail above. The principle and practice of the present invention were illustrated by specific Examples above. Description of the Examples is only used to facilitate the understanding of the methods and key concepts of the present invention.

## Claims

1. A method for purifying crude liraglutide obtained from solid-phase synthesis, which is **characterized by** comprising the following steps:
Step 1: a solution of crude liraglutide is obtained by dissolving crude liraglutide obtained from solid-phase synthesis in aqueous acetonitrile solution;
Step 2: the solution of crude liraglutide is subjected to a first HPLC purification using octylsilane bonded silica as stationary phase, and using aqueous isopropanol solution containing 0.1-0.2% trifluoroacetic acid as mobile phase A and acetonitrile containing 0.1-0.2% trifluoroacetic acid as mobile phase B eluting at a linear gradient from 20-40% B to 40-60% B, and target peak is collected as the first fraction;
Step 3: the first fraction is subjected to a second HPLC purification using cyanosilane bonded silica as stationary phase, and using 0.05-0.15% (mass concentration) aqueous perchloric acid solution as mobile phase A and 0.05-0.15% (mass concentration) perchloric acid in acetonitrile as mobile phase B eluting at a linear gradient from 40% B to 70% B, and target peak is collected as the second fraction;
Step 4: the second fraction is subjected to a third HPLC purification using octylsilane bonded silica as stationary phase, and using 0.01-0.06% (mass concentration) aqueous ammonia solution as mobile phase A and acetonitrile of chromatographic grade as mobile phase B eluting at a linear gradient from 30% B to 60% B, and target peak is collected as the third fraction;
Step 5: purified peptide is obtained from the third fraction by rotatory evaporation under reduced presser and lyophilization.

2. The purification method according to claim 1, which is **characterized in that** the volume ratio between acetonitrile and water in the aqueous acetonitrile solution is 10-30 : 70-90.

3. The purification method according to claim 1, which is **characterized in that** the volume ratio between isopropanol and water in the aqueous isopropanol solution is 15-35 : 65-85.

4. The purification method according to claim 1, which is **characterized in that** flow rate for the first, the second or the third HPLC purification in Step 2, 3, or 4 is 55-2000 ml/min.

5. The purification method according to claim 1, which is **characterized in that** flow rate for the first, the second or the third HPLC purification in Step 2, 3, or 4 is 55-500 ml/min.

6. The purification method according to claim 1, which is **characterized in that** the duration for the linear gradient elution in Step 2 or 3 is 40 min.

7. The purification method according to claim 1, which is **characterized in that** the duration for the linear gradient elution in Step 4 is 30 min.

8. The purification method according to claim 1, which is **characterized in that** the concentration after rotatory evaporation under reduced pressure in Step 5 is 50-70 mg/ml.

9. The purification method according to claim 1, which is **characterized in that** the solid-phase synthesis is performed according to the following steps: in the presence of activating agent system, coupling solid-phase support resin with N-terminal Fmoc-protected glycine to obtain Fmoc-Gly-resin; according to backbone sequence of liraglutide, sequentially coupling amino acids with N-terminal Fmoc protection and side chain protection using solid-phase synthesis method, with Alloc protection for the side chain of lysine; removing the protective group Alloc from the side chain of lysine; coupling palmitoyl-Gllu-OtBu to the side chain of lysine by solid-phase synthesis method; obtaining crude liraglutide after cleavage, and removal of protective group and resin.

## Patentansprüche

1. Verfahren zum Aufreinigen von unverarbeitetem Liraglutid, das aus einer Festphasensynthese gewonnen wird, wobei das Verfahren durch die folgenden Schritte gekennzeichnet ist:
Schritt 1: eine Lösung von unverarbeitetem Liraglutid wird erhalten durch Auflösen von unverarbeitetem Liraglutid, das aus einer Festphasensynthese gewonnen wird, in einer wässrigen Acetonitrillösung;
Schritt 2: die Lösung von unverarbeitetem Liraglutid wird einer ersten HPLC-Aufreinigung unter Verwendung von Octylsilan-gebundener Kieselerde als stationäre Phase unterzogen und unter Verwendung einer wässrigen Isopropanollösung, die 0,1 - 0,2% Trifluoressigsäure als mobile Phase A und Acetonitril, das 0,1 - 0,2% Trifluoressigsäure als mobile Phase B enthält, eluierend mit einem linearen Gradienten von 20 - 40% B zu 40 - 60% B, und wobei der Target Peak als erster Teil aufgenommen wird;
Schritt 3: der erste Teil wird einer zweiten HPLC-Aufreinigung unter Verwendung von Cyanosilan-gebundener Kieselerde als stationäre Phase unterzogen und unter Verwendung von 0,05 - 0,15% (Massenkonzentration) wässriger Perchlorsäurelösung als mobile Phase A und von 0,05 - 0,15% (Massenkonzentration) Perchlorsäure in Acetonitril als mobile Phase B, eluierend mit einem linearen Gradienten von 40% B bis 70% B, und wobei der Target Peak als zweiter Teil aufgenommen wird;
Schritt 4: der zweite Teil wird einer dritten HPLC-Aufreinigung unter Verwendung von Octylsilan-gebundener Kieselerde als stationäre Phase unterzogen und unter Verwendung von 0,01 - 0,06% (Massenkonzentration) wässriger Ammoniaklösung als mobile Phase A und von Acetonitril chromatographischer Qualität als mobile Phase B, eluierend mit einem linearen Gradienten von 30% B bis 60% B, und wobei der Target Peak als dritter Teil aufgenommen wird;
Schritt 5: aufgereinigtes Peptid wird erhalten aus dem dritten Teil durch Rotationsverdampfung unter reduziertem Druck und Lyophilisation.

2. Aufreinigungsverfahren nach Anspruch 1, das **dadurch gekennzeichnet ist, dass** das Volumenverhältnis zwischen Acetonitril und Wasser in der wässrigen Acetonitrillösung 10-30 : 70-90 ist.

3. Aufreinigungsverfahren nach Anspruch 1, das **dadurch gekennzeichnet ist, dass** das Volumenverhältnis zwischen Isopropanol und Wasser in der wässrigen Isopropanollösung 15-35 : 65:85 ist.

4. Aufreinigungsverfahren nach Anspruch 1, das **dadurch gekennzeichnet ist, dass** die Strömungsgeschwindigkeit für die erste, die zweite oder die dritte HPLC-Aufreinigung in Schritt 2, 3 oder 4 55 - 2000 ml/min beträgt.

5. Aufreinigungsverfahren nach Anspruch 1, das **dadurch gekennzeichnet ist, dass** die Strömungsgeschwindigkeit für die erste, die zweite oder die dritte HPLC-Aufreinigung in Schritt 2, 3 oder 4 55 - 500 ml/min beträgt.

6. Aufreinigungsverfahren nach Anspruch 1, das **dadurch gekennzeichnet ist, dass** die Dauer für die Eluierung mit linearem Gradienten in Schritt 2 oder 3 40 Minuten beträgt.

7. Aufreinigungsverfahren nach Anspruch 1, das **dadurch gekennzeichnet ist, dass** die Dauer für die Eluierung mit linearem Gradienten in Schritt 4 30 Minuten beträgt.

8. Aufreinigungsverfahren nach Anspruch 1, das **dadurch gekennzeichnet ist, dass** die Konzentration nach der Rotationsverdampfung unter reduziertem Druck in Schritt 5 50 - 70 mg/ml beträgt.

9. Aufreinigungsverfahren nach Anspruch 1, das **dadurch gekennzeichnet ist, dass** die Festphasensynthese gemäß den folgenden Schritten durchgeführt wird: in Gegenwart eines Aktivierungsmittelsystems wird ein Trägerharz mit fester Phase mit N-terminalem Fmoc-geschütztem Glycin gebunden, um Fmoc-Gly-Harz zu erhalten; gemäß der Hauptsequenz von Liraglutid werden Aminosäuren sequentiell mit N-terminalem Fmoc-Schutz und Nebenkettenschutz unter Verwendung von Festphasensynthesemethoden gebunden, mit Alloc-Schutz für die Nebenkette von Lysin; Entfernen der Alloc-Schutzgruppe von der Nebenkette von Lysin; Binden von Palmitoyl-Gllu-OtBu an die Nebenkette von Lysin durch eine Festphasensynthesemethode; Erhalten von unverarbeitetem Liraglutid nach Spaltung und Entfernung von Schutzgruppe und Harz.

## Revendications

1. Procédé de purification de liraglutide brut obtenu à partir d'une synthèse en phase solide, qui est **caractérisé en ce qu'**il comprend les étapes suivantes :
Étape 1 : une solution de liraglutide brut est obtenue en dissolvant du liraglutide brut obtenu à partir d'une synthèse en phase solide dans une solution d'acétonitrile aqueux ;
Étape 2 : la solution de liraglutide brut est soumise à une première purification par CLHP en utilisant de la silice liée à l'octylsilane en tant que phase stationnaire, et en utilisant une solution d'isopropanol aqueux contenant de l'acide trifluoroacétique à 0,1-0,2% en tant que phase mobile A et de l'acétonitrile contenant de l'acide trifluoroacétique à 0,1-0,2% en tant que phase mobile B éluant à un gradient linéaire compris entre 20-40% B et 40-60% B, et un pic cible est recueilli en tant que première fraction ;
Étape 3 : la première fraction est soumise à une seconde purification par CLHP en utilisant de la silice liée à l'octylsilane en tant que phase stationnaire, et en utilisant une solution d'acide perchlorique aqueux à 0,05-0,15% (concentration massique) en tant que phase mobile A et de l'acide perchlorique à 0,05-0,15% (concentration massique) dans de l'acétonitrile en tant que phase mobile B éluant à un gradient linéaire compris entre 40% B et 70% B, et un pic cible est recueilli en tant que seconde fraction ;
Étape 4 : la seconde fraction est soumise à une troisième purification par CLHP en utilisant de la silice liée à l'octylsilane en tant que phase stationnaire, et en utilisant une solution d'ammoniac aqueux à 0,01-0,06% (concentration massique) en tant que phase mobile A et de l'acétonitrile de qualité chromatographique % en tant que phase mobile B éluant à un gradient linéaire compris entre 30% B et 60% B, et un pic cible est recueilli en tant que troisième fraction ;
Étape 5 : un peptide purifié est obtenu à partir de la troisième fraction par évaporation rotative sous pression réduite et par lyophilisation.

2. Procédé de purification selon la revendication 1, qui est **caractérisé en ce que** le rapport volumique entre l'isopropanol et l'eau dans la solution d'acétonitrile aqueux est 10-30 : 70-90.

3. Procédé de purification selon la revendication 1, qui est **caractérisé en ce que** le rapport volumique entre l'isopropanol et l'eau dans la solution d'isopropanol aqueux est 10-30 : 65-85.

4. Procédé de purification selon la revendication 1, qui est **caractérisé en ce que** le débit pour la première, la seconde ou la troisième purification par CLHP dans l'Étape 2, 3 ou 4 est compris entre 55 et 2000 mL/min.

5. Procédé de purification selon la revendication 1, qui est **caractérisé en ce que** le débit pour la première, la seconde ou la troisième purification par CLHP dans l'Étape 2, 3 ou 4 est compris entre 55 et 500 mL/min.

6. Procédé de purification selon la revendication 1, qui est **caractérisé en ce que** la duration de l'élution à gradient linéaire dans l'Étape 2 ou l'Étape 3 est de 40 min.

7. Procédé de purification selon la revendication 1, qui est **caractérisé en ce que** la duration de l'élution à gradient linéaire dans l'Étape 4 est de 30 min.

8. Procédé de purification selon la revendication 1, qui est **caractérisé en ce que** la concentration après évaporation rotative sous pression réduite dans l'Étape 5 est comprise entre 50 et 70 mg/mL.

9. Procédé de purification selon la revendication 1, qui est **caractérisé en ce que** la synthèse en phase solide est effectuée selon les étapes suivantes : en présence d'un système d'agent d'activation, coupler une résine de support en phase solide avec une glycine protégé par Fmoc au N terminal pour obtenir Fmoc-Gly-résine ; selon une séquence de squelette de liraglutide, coupler séquentiellement des acides aminés avec une protection Fmoc au N terminal et une protection de chaine latérale en utilisant un procédé de synthèse en phase solide, avec une protection Alloc pour la chaine latérale de lysine ; éliminer le groupe de protection Alloc de la chaine latérale de lysine ; coupler palmitoyl-Gllu-OtBu à la chaine latérale de lysine par un procédé de synthèse en phase solide ; obtenir un liraglutide brut après clivage, et élimination du groupe de protection et de la résine.
